(19) Europäisches Patentamt
European Patent Office
Office européen des brevets

(11) Publication number: **0 281 266 B1**

(12) **EUROPEAN PATENT SPECIFICATION**

(45) Date of publication of patent specification: **16.12.92**

(51) Int. Cl.$^5$: **C07C 229/60**

(21) Application number: **88301225.4**

(22) Date of filing: **15.02.88**

(54) **3-Amino-2,4,5-trifluorobenzoic acid and its manufacture.**

(30) Priority: **13.02.87 JP 29582/87**

(43) Date of publication of application:
**07.09.88 Bulletin 88/36**

(45) Publication of the grant of the patent:
**16.12.92 Bulletin 92/51**

(84) Designated Contracting States:
**DE ES FR GB IT**

(56) References cited:
**EP-A- 0 266 512**
**DE-A- 3 631 906**
**GB-A- 915 587**
**GB-A- 991 537**

(73) Proprietor: **NIPPON SHOKUBAI CO., LTD.**
**1-1, Koraibashi, 4-chome**
**Chuo-ku, Osaka-shi(JP)**

(72) Inventor: **Hirota, Koichi**
**c/o Kawazuraryo 4-52 Nakanoshima-cho**
**Suita-shi Osaka-fu(JP)**
Inventor: **Kaieda, Osamu**
**406-59 Todaiji Shimamoto-cho**
**Mishima-gun Osaka-fu(JP)**
Inventor: **Takaya, Tsuguo**
**13-29 Ohira 2-chome**
**Ohtsu-shi Shiga-ken(JP)**

(74) Representative: **Sheard, Andrew Gregory et al**
**Kilburn & Strode 30, John Street**
**London WC1N 2DD(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

The present invention relates to 3-amino-2,4,5-trifluorobenzoic acid and a method for its manufacture.

3-amino-2,4,5-trifluorobenzoic acid is a novel compound, as the compound itself and a method for its manufacture have not yet been reported.

An object of the present invention is, accordingly, to provide 3-amino-2,4,5-trifluorobenzoic acid and a method for its manufacture.

The above-mentioned object can be attained by providing 3-amino-2,4,5-trifluorobenzoic acid.

The object can also be attained by a method for manufacturing 3-amino-2,4,5-trifluorobenzoic acid by subjecting 4-amino-3,5,6-trifluorophthalonitrile to heat in the presence of an acid.

3-Amino-2,4,5-trifluorobenzoic acid is useful as a starting material or intermediate for synthesizing pharmaceutical, especially antimicrobial, agents. 3-Amino-2,4,5-trifluorobenzoic acid is also useful as a monomer for preparation of polyamides, because it has both amino and carboxyl groups in the molecule.

The single figure in the accompanying drawing is an infrared absorption spectrum chart of 3-amino-2,4,5-trifluorobenzoic acid.

3-Amino-2,4,5-trifluorobenzoic acid can be manufactured by heating 4-amino-3,5,6-trifluorophthalonitrile in the presence of an acid. As the acid, any acid which is usually used in general hydrolysis such as an inorganic acid, e.g. sulphuric acid, hydrogen chloride or phosphoric acid can be used; sulphuric acid is preferred. The acid is usually used as aqueous solution.

Typically, 3-amino-2,4,5-trifluorobenzoic acid can be manufactured by heating 4-amino-3,5,6-trifluorophthalonitrile in an aqueous sulphuric acid solution at a temperature of 90° to 170°C.

It is assumed, although the applicants do not wish to be bound by this theory, that the reaction proceeds by hydrolysis and decarboxylation at the same time as shown in equation I to form 3-amino-2,4,5-trifluorobenzoic acid.

Equation I:

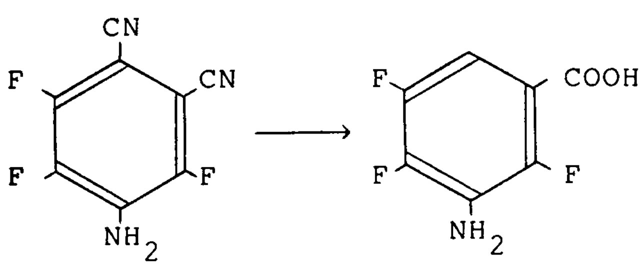

The concentration of the aqueous acid solution may be 20 to 90% by weight, preferably 40 to 70% by weight. If the concentration of the acid exceeds 90% by weight, a sudden reaction is apt to occur and to generate heat, so it is dangerous. If the concentration is less than 20% by weight, the reaction rate decreases and productivity signficantly decreases, so it is not preferred. Therefore, it is preferable to select an appropriate concentration of the acid.

The preferred reaction temperature is 90° to 170°C, preferably 110° to 150°C. If the temperature exceeds 170°C, a sudden reaction occurs and generates heat, so it is dangerous. If the temperature is less than 90°C, the reaction rate and productivity decrease, so it is not preferred.

It is usually preferable that the reaction is carried out under reflux and under normal pressure. When the reaction is carried out under reflux, the reaction temperature depends especially on the acid concentration, but the reaction may be carried out under high or reduced pressure in order to avoid the dependence on the acid concentration. Further, when the reaction is carried out under normal pressure, it is not always carried out under reflux, and it may be carried out by controlling it at a lower temperature.

The reaction time is not limiting; it is preferably from 3 to 30 hours, more preferably 10 to 20 hours.

The feed concentration of 4-amino-3,5,6-trifluorophthalonitrile is preferred to be from 100 to 1,000 parts by weight, preferably 200 to 700 parts by weight of the aqueous acid solution per 100 parts by weight of 4-amino-3,5,6-trifluorophthalonitrile. The reaction may be initiated after feeding 4-amino-3,5,6-trifluorophthalonitrile and the aqueous acid solution at the same time into a reaction zone. Further, the reaction may safely be carried out by controlling the reaction as follows:

(a) adding 4-amino-3,5,6-trifluorophthalonitrile continuously or intermittently to the aqueous acid solution

in a reaction zone;

(b) adding the acid or the aqueous acid solution continuously or intermittently to an aqueous slurry solution of 4-amino-3,5,6-trifluorophthalonitrile in a reaction zone; or

(c) controlling the reaction rate by feeding both 4-amino-3,5,6-trifluorophthalonitrile and a low concentration of an aqueous acid solution to a reaction zone to initiate the reaction, and then adding the acid or the aqueous acid solution continuously or intermittently to the mixture as the reaction proceeds to vary the acid concentration and increase the reaction temperature step by step. In such case, the concentration of acid to be fed prior to the initiation of the reaction is preferably 10 to 70% by weight, and the concentration of acid to be added is preferably 40 to 98% by weight. The reaction is preferably carried out by initiating the reaction at a temperature of from 90° to 140°C and then elevating the reaction temperature to 120° to 170°C by adding the acid gradually along with the proceeding of the reaction.

In any method an inert organic solvent (for example a ketone such as acetone or methyl ethyl ketone, a nitrile such as acetonitrile or benzonitrile, or an aprotic polar solvent such as dimethyl formamide or sulpholane), may be used in the reaction system.

Further, although 3-amino-2,4,5-trifluorobenzoic acid is preferably manufactured by conducting hydrolysis and decarboxylation of the starting 4-amino-3,5,6-triflurophthalonitrile at the same time because of the simple process involved, 4-amino-3,5,6-triflurophthalonitrile may be prior hydrolyzed to produce 4-amino-3,5,6-trifluorophthalic acid and then decarboxylated it to produce 3-amino-2,4,5-trifluorobenzoic acid. Further, 4-amino-3,5,6-trifluorophthalic acid may be prior esterified to produce ester such as dimethyl 4-amino-3,5,6-trifluorophthalate and then decarboxylated it to produce 3-amino-2,4,5-trifluorobenzoic acid.

4-Amino-3,5,6-trifluorophthalonitrile as to the starting material in the present invention is a known compound and can easily be obtained by the method disclosed in Ishikawa et al, Yuki Gosei Kagaku Kyokaishi, 29, (8), 794 (1971) or Birchall et al, Journal of the Chemical Society (C), 1970, 456 (1970).

A method for manufacturing the compound of the present invention will be explained by the following examples in detail; results of analysis for identifying the novel compound are also described.

The invention is not limited by the examples.

Example 1

Into a 200ml four-necked separable flask having a stirrer, a thermometer and a Dimroth condenser, 200g of 60% by weight of aqueous sulphuric acid solution was fed and further 30.0g (0.152 mole) of 4-amino-3,5,6-trifluorophthalonitrile was charged, then heated to elevate the temperature. The reaction was carried out with adequate stirring at a temperature of 128° to 130°C under reflux for 3 hours.

After cooling, the reaction mixture was poured into ice water, and then 3-amino-2,4,5-trifluorobenzoic acid was extracted from the aqueous solution containing 3-amino-2,4,5-trifluorobenzoic acid by using 400ml of isopropyl ether. The isopropyl ether layer thus obtained was dried by anhydrous magnesium sulphate and then 27.7g (0.145 mole, 95.2 mol% of yield to 4-amino-3,5,6-trifluorophthalonitrile) of 3-amino-2,4,5-trifluorobenzoic acid was obtained.

The compound was further purified by recrystallization with benzene-ethanol mixture solvent, and the purified 3-amino-2,4,5-trifluorobenzoic acid was analyzed to obtain physical properties and decided the structure.

Melting point: 140° - 141°C

Elemental analysis:

| | C(%) | H(%) | N(%) | F(%) |
|---|---|---|---|---|
| Theoretical | 43.99 | 2.11 | 7.33 | 29.82 |
| Found | 44.12 | 2.13 | 7.41 | 29.96 |

| Infrared spectrum (KBr tablet, unit: $cm^{-1}$) | |
|---|---|
| 3420, 3335 | (amino nu-N-H) |
| 2500-3200 | (hydroxy nu-O-H) |
| 1695 | (carbonyl nu > C = O) |

The infrared absorption spectrum is shown in the figure.

$^{19}F$ NMR (solvent:acetone-$d^6$, inner standard substance: $CF_3COOH$) ppm

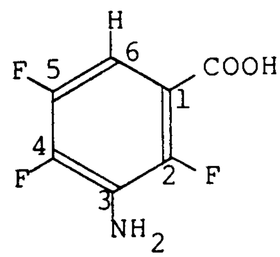

$F_2$ delta -55.87, ddd (J = 19.5, 13.4, 6.4)

$F_4$ delta -73.91, ddd (J = 19.5, 20.4, 8.8)

$F_5$ delta -67.90, ddd (J = 13.4, 20.4, 10.7)

Example 2

A similar method to Example 1 was carried out except that the concentration of sulphuric acid was 45% by weight, the reaction temperature was 120 ° C and the reaction time was 12 hours.

As a result, 28.1g (96.6 mol% of yield to 4-amino-3,5,6-trifluorophthalonitrile) of 3-amino-2,4,5-trifluorobenzoic acid was obtained.

Example 3

Into a 200ml four-necked separable-flask having a stirrer, a thermometer and a Dimroth condenser, 200g of 60% by weight of aqueous sulphuric acid solution was fed, heated under stirring and maintained at a temperature of 130 ° C. 30.0g (0.152 mole) of 4-amino-3,5,6-trifluorophthalonitrile was added to the solution gradually for 3 hours, and then subjected to reaction for 2 hours.

Then an extraction operation similar to Example 1 was carried out to obtain 27.2g (0.142 mole, 93.5 mol% of yield to 4-amino-3,5,6-trifluorophthalonitrile) of 3-amino-2,4,5-trifluorobenzoic acid.

Example 4

Into a 200ml four-necked separable flask having a stirrer, a thermometer and a Dimroth condenser, 76.3g of water and 30.0g (0.152 mole) of 4-amino-3,5,6-trifluorophthalonitrile were fed, and the mixture thus obtained was heated under stirring and maintained at a temperature of 100 ° C. Into the mixture, 123.7g of 97% by weight of concentrated sulphuric acid was dropped for 2 hours to elevate the temperature from 100 ° C to 128 ° C, and further the reaction was continued for 3 hours at a temperature of 128 to 130 ° C.

Then an extraction operation similar to Example 1 was carried out to obtain 27.5g (0.144 mole, 94.5 mol% of yield to 4-amino-3,5,6-trifluorophthalonitrile) of 3-amino-2,4,5-trifluorobenzoic acid.

Example 5

Into a 200ml four-necked separable-flask having a stirrer, a thermometer and a Dimroth condenser, 140g of 55% aqueous sulphuric acid solution was fed, and 50g (0.254 mole) of 4-amino-3,5,6-trifluorophthalonitrile was fed, then the temperature of the mixture thus obtained was elevated and subjected to reaction under adequate stirring and refluxing at a temperature of 128 to 129 ° C for 7 hours. Then 9.2g of 97% by weight of concentrated sulphuric acid was added gradually during the reaction and subjected to reaction under refluxing at a temperature of 131 to 134 ° C for 4 hours, and further 8g of 97% by weight of concentrated sulphuric acid was added gradually under refluxing at a temperature of 134 ° C to 135 ° C for 4 hours.

Then an extraction operation similar to Example 1 was carried out to obtain 46.8g (0.246 mole, 96.5 mol% of yield to 4-amino-3,5,6-trifluorophthalonitrile).

**Claims**

1. 3-Amino-2,4,5-trifluorobenzoic acid.

2. A method for manufacturing 3-amino-2,4,5-trifluorobenzoic acid which comprises subjecting 4-amino-3,5,6-trifluorophthalonitrile to heat in the presence of an acid.

3. A method according to claim 2, wherein the acid is used as an aqueous solution.

4. A method according to claim 2 or 3, wherein the heating is carried out at a temperature of from 90° to 170° C.

5. A method according to claim 2, 3 or 4, wherein the acid is an inorganic acid.

6. A method according to any one of claims 2 to 5, wherein the acid is sulphuric acid, hydrogen chloride or phosphoric acid.

7. A method according to claim 6, wherein the acid is sulphuric acid.

8. A method according to any one of claims 2 to 7, wherein the acid is used in a concentration of 20 to 90% by weight.

9. A method according to claim 3, wherein the aqueous acid solution is used in an amount of 100 to 1,000 parts by weight per 100 parts by weight of 4-amino-3,5,6-trifluorophthalonitrile.

10. A method according to claim 3, wherein the 4-amino-3,5,6-trifluorophthalonitrile and the aqueous acid solution are fed to a reaction zone at the same time.

11. A method according to claim 3, wherein the 4-amino-3,5,6-trifluorophthalonitrile is fed continuously or intermittently to the aqueous acid solution in a reaction zone to conduct the reaction.

12. A method according to claim 2 or 3, wherein the acid or the aqueous acid solution is fed continuously or intermittently to an aqueous slurry solution of 4-amino-3,5,6-trifluorophthalonitrile in a reaction zone to conduct the reaction.

13. A method according to claim 2 or 3, wherein 4-amino-3,5,6-trifluorophthalonitrile and a low concentration of an aqueous acid solution are fed to a reaction zone at the same time to initiate the reaction, and then said acid or said aqueous acid solution is fed continuously or intermittently to conduct the reaction.

## Patentansprüche

1. 3-Amino-2,4,5-trifluorbenzoesäure.

2. Verfahren zur Herstellung von 3-Amino-2,4,5-trifluorbenzoesäure, bei dem man 4-Amino-3,5,6-trifluorphthalonitril in Gegenwart einer Säure erwärmt.

3. Verfahren gemäß Anspruch 2, wobei man die Säure als eine wässrige Lösung einsetzt.

4. Verfahren gemäß Anspruch 2 oder 3, wobei man die Erwärmung bei einer Temperatur von 90°C bis 170°C durchführt.

5. Verfahren gemäß Anspruch 2, 3 oder 4, wobei die Säure eine anorganische Säure ist.

6. Verfahren gemäß irgendeinem der Ansprüche 2 bis 5, wobei die Säure Schwefelsäure, Salzsäure oder Phosphorsäure ist.

7. Verfahren gemäß Anspruch 6, wobei die Säure Schwefelsäure ist.

8. Verfahren gemäß irgendeinem der Ansprüche 2 bis 7, wobei man die Säure in einer Konzentration von 20 bis 90 Gewichtsprozent verwendet.

9. Verfahren gemäß Anspruch 3, wobei man die wässrige Säurelösung in einer Menge von 100 bis 1000 Gewichtsteilen je 100 Gewichtsteile 4-Amino-3,5,6-trifluorphthalonitril benutzt.

10. Verfahren gemäß Anspruch 3, wobei man 4-Amino-3,5,6-trifluorphthalonitril und die wässrige Säurelösung gleichzeitig in ein Reaktionsgefäß einfüllt.

11. Verfahren gemäß Anspruch 3, wobei man 4-Amino-3,5,6-trifluorphthalonitril kontinuierlich oder stoßweise zu der wässrigen Säurelösung in ein Reaktionsgefäß zugibt, um die Reaktion durchzuführen.

12. Verfahren gemäß Anspruch 2 oder 3, wobei man die Säure oder die wässrige Säurelösung kontinuierlich oder stoßweise zu einer wässrigen Schlämmlösung von 4-Amino-3,5,6-trifluorphthalonitril in ein Reaktionsgefäß zugibt, um die Reaktion durchzuführen.

13. Verfahren gemäß Anspruch 2 oder 3, wobei man 4-Amino-3,5,6-trifluorphthalonitril und eine wässrige Säurelösung mit niedriger Konzentration gleichzeitig in ein Reaktionsgefäß einfüllt, um die Reaktion zu starten, und anschließend die Säure oder die wässrige Säurelösung kontinuierlich oder stoßweise zugibt, um die Reaktion durchzuführen.

**Revendications**

1. Acide 3-amino-2,4,5-trifluorobenzoïque.

2. Procédé pour la fabrication d'acide 3-amino-2,4,5-trifluorobenzoïque qui comprend l'opération consistant à soumettre le 4-amino-3,5,6-trifluorophtalonitrile à la chaleur en présence d'un acide.

3. Procédé selon la revendication 2, dans lequel l'acide est utilisé sous forme de solution aqueuse.

4. Procédé selon la revendication 2 ou 3, dans lequel le chauffage est conduit à une température allant de 90 jusqu'à 170 ° C.

5. Procédé selon la revendication 2, 3 ou 4 dans lequel l'acide est un acide inorganique.

6. Procédé selon l'une quelconque des revendications 2 à 5, dans lequel l'acide est l'acide sulfurique, un chlorure hydrogéné ou un acide phosphorique.

7. Procédé selon la revendication 6, dans lequel l'acide est l'acide sulfurique.

8. Procédé selon l'une quelconque des revendications 2 à 7 dans lequel l'acide est utilisé dans une concentration de 20 à 90 % en poids.

9. Procédé selon la revendication 3, dans lequel la solution acide aqueuse est utilisée dans une quantité de 100 à 1000 parties en poids pour 100 parties en poids de 4-amino-3,5,6-trifluorophtalonitrile.

10. Procédé selon la revendication 3, dans lequel le 4-amino-3,5,6-trifluorophtalonitrile et la solution acide aqueuse sont alimentés en même temps dans une zone réactionnelle.

11. Procédé selon la revendication 3, dans lequel le 4-amino-3,5,6-trifluorophtalonitrile est alimenté en continu ou par intermittence à la solution acide aqueuse dans une zone réactionnelle pour conduire la réaction.

12. Procédé selon la revendication 2 ou 3, dans lequel on alimente l'acide ou la solution acide aqueuse en continu ou par intermittence à une solution de bouillie aqueuse de 4-amino-3,5,6-trifluorophtalonitrile dans une zone réactionnelle pour conduire la réaction.

13. Procédé selon la revendication 2 ou 3, dans lequel le 4-amino-3,5,6-trifluorophtalonitrile et une faible

concentration d'une solution acide aqueuse sont alimentés en même temps dans une zone réactionnelle pour faire démarrer la réaction, on alimente ensuite en continu ou par intermittence l'acide ou la solution acide aqueuse pour conduire à la réaction.

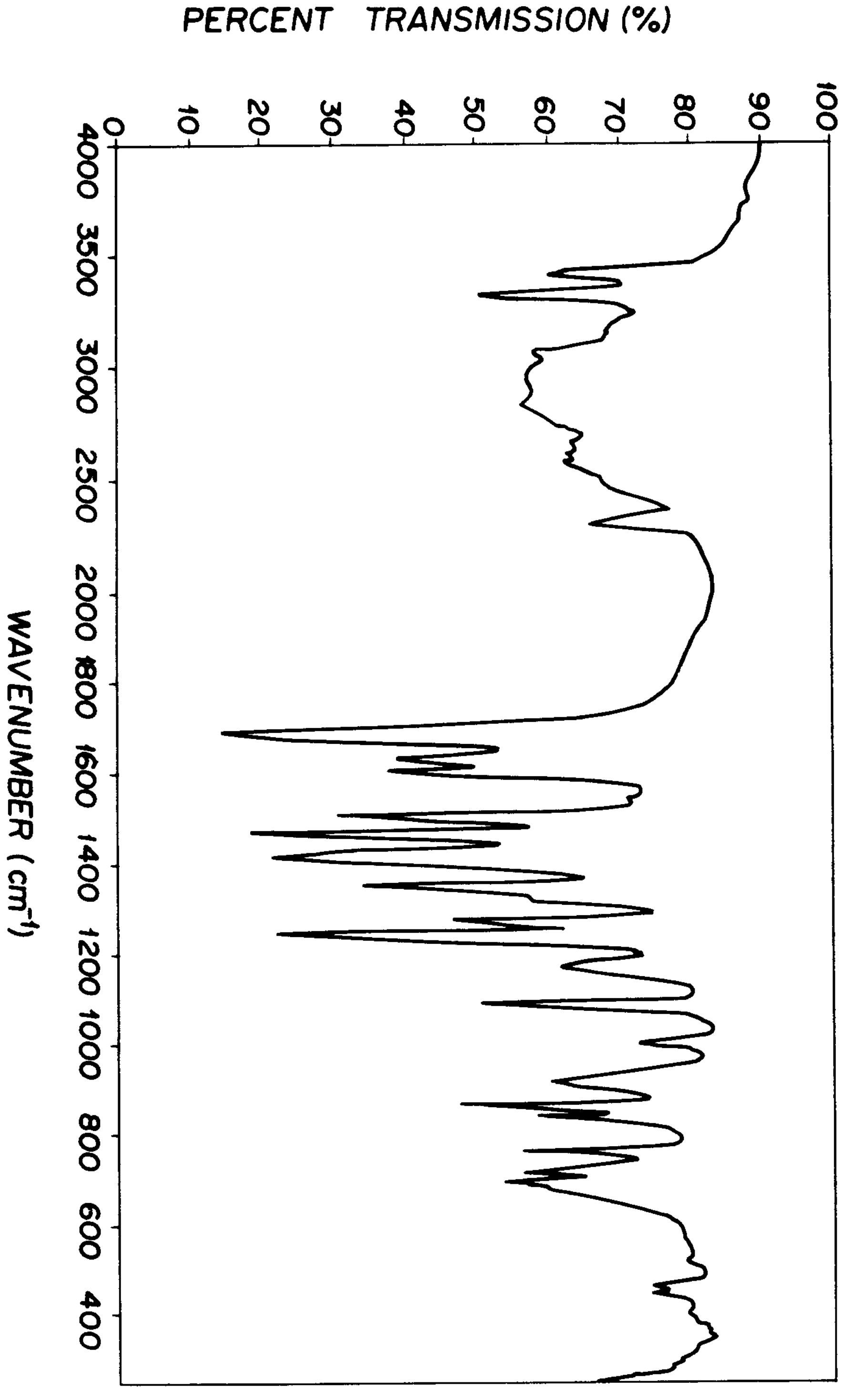
PERCENT TRANSMISSION (%)
0
10
20
30
40
50
60
70
80
90
100
4000
3500
3000
2500
2000
1800
1600
1400
1200
1000
800
600
400
WAVENUMBER (cm-1)